# EUROPÄISCHE PATENTSCHRIFT

(11) **EP 1 037 875 B1**
(45) Veröffentlichungstag und Bekanntmachung des Hinweises auf die Patenterteilung: **28.09.2005**
(21) Anmeldenummer: 98963474.6
(22) Anmeldetag: 07.12.1998
(51) Int. Cl.: C07D 207/22, C07D 211/68, C07B 43/00

(54) **VERFAHREN ZUR HERSTELLUNG VON SALZEN CYCLISCHER AMIDINE**
METHOD FOR PRODUCING SALTS OF CYCLIC AMIDINES
PROCEDE POUR LA PRODUCTION DE SELS D'AMIDINES CYCLIQUES

(30) Priorität: 08.12.1997 DE 19754322
(43) Veröffentlichungstag der Anmeldung: 27.09.2000
(73) Patentinhaber: Degussa AG, 40474 Düsseldorf (DE)
(72) Erfinder: MOST, Dieter, D-63486 Bruchköbel (DE); DRAUZ, Karlheinz, D-63579 Freigericht (DE)
(86) Internationale Anmeldenummer: PCT/EP1998/007373
(87) Internationale Veröffentlichungsnummer: WO 1999/029665

(56) Entgegenhaltungen:
- WO-A-94/22828
- J. BARLUENGA ET AL.: "Tandem amidomercuration-reductive alkylation of alkenes. One-pot synthesis of amidic 1,4-bifunctionalized compounds." SYNTHESIS., Nr. 10, 1984, Seiten 831-832, XP002099072 STUTTGART DE in der Anmeldung erwähnt

## Beschreibung

Die vorliegende Erfindung betrifft ein Verfahren zur Herstellung von Verbindungen der allgemeinen Formel (I) 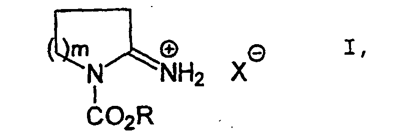 wobei X^{⊖} ein anorganisches Anion, m = 1, 2 oder 3 und R einen linearen (C₁-C₄)-Alkylrest darstellt.

Verbindungen des o. g. Strukturtyps sind vorteilhafte Intermediate zur Herstellung von bioaktiven Wirkstoffen, wie sie aus US 4,213,773 und WO 94/22828 bekannt sind.

In WO 94/22828 wird die Möglichkeit der Zyklisierung von N-(4-Cyanobutyl)-carbaminsäurealkylestern mittels eines wasserfreien Halogenwasserstoffs beschrieben. Dort werden bezüglich dieser Reaktion keine Ausbeuten genannt. Zur Herstellung des N-(4-Cyanobutyl)-carbaminsäurealkylesters wird auf eine Veröffentlichung aus Synthesis 1984, 831 verwiesen. Mit dem dort genannten Verfahren erhält man diese Verbindung allerdings nur in max. 32 % Ausbeute. D. h., daß die Gesamtausbeute der Herstellung von (I), im Falle von m = 2 und R = Me, nach dem Stand der Technik max. 32 % betragen kann. Zudem kommt bei diesem Verfahren ein Quecksilbersalz zum Einsatz, was großtechnisch gesehen aus ökologischen Gründen unmöglich zu realisieren ist. Zur Herstellung von N-(4-Cyanobutyl)-carbaminsäuremethylester ist es laut Synthesis-Verfahren notwendig, das einzusetzende Ethen durch eine unter Rückfluß befindliche flüssige Lösung aus Methylcarbamat und Quecksilbernitrat in Dichlormethan zu leiten. Abgesehen davon, daß Ethen dazu neigt, in Kontakt mit Lewis-Säuren oder Lewis-Basen sowie Radikalen zu polymerisieren, ist die bei großtechnischen Verfahren notwendige Lagerung dieser risikoreichen Chemikalie äußerst bedenklich.

Aufgabe der Erfindung war es somit, ein verglichen mit dem Stand der Technik in Bezug auf ökonomische wie ökologische Gesichtspunkte besseres Verfahren zur Herstellung der Verbindungen der allgemeinen Formel (I) zu finden, welches im großtechnischen Maßstab einen risikoloseren stabilen Prozeßverlauf gestattet.

Diese und nicht näher genannte weitere Aufgaben werden durch ein Verfahren nach den Merkmalen des kennzeichnenden Teils des Anspruches 1 gelöst. Besonders vorteilhafte Ausgestaltungen des Verfahrens sind Gegenstand der von Anspruch 1 abhängigen Unteransprüche.

Dadurch, daß man eine entsprechende enantiomerenreine oder racemische ω-Amino-α-aminosäure selektiv an seiner ω-Aminofunktion zu Verbindungen der allgemeinen Formel (II) 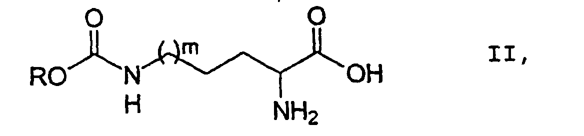 umsetzt, die so erhaltenen Verbindungen (II) zu Substanzen der allgemeinen Formel (III) 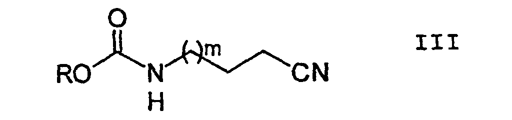 oxidativ decarboxyliert und die Verbindung (III) unter Wasserausschluß in Gegenwart einer Säure unter Ringschluß zum Salz der allgemeinen Formel (I) 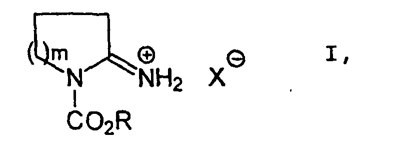 umsetzt, gelangt man in sehr guten Gesamtausbeuten von >70 % ohne den Anfall von ökologisch bedenklichen Abfällen in einem robusten Verfahren zu den gewünschten Verbindungen. In diesem Verfahren ist weiterhin vorteilhaft, daß weitgehend alle Lösungsmittelströme im Kreis gefahren werden können, wodurch die unweigerlich anfallende Abfallmenge auf ein maximal mögliches Maß reduziert wird.

Besonders vorteilhaft ist es, die entsprechende ω-Amino-α-aminosäure oder eine diese Carbonsäure erzeugende Vorstufe unter basischen Bedingungen mit einem Reagenz der allgemeinen Formel (IV) 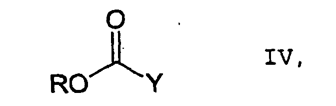 worin Y = Cl, Br oder OMe oder 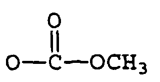 und R = (C₁-C₄)-Alkyl, welches bevorzugt linear vorliegt, oder Aryl ist, zu Verbindungen der allgemeinen Formel (II) umzusetzen. Als ω-Amino-α-aminosäuren erzeugende Vorstufen kommen dabei deren kommerziell erhältliche Hydrochloride, Hydrate oder die Verbindungen in Form ihrer freien Basen als ca. 50%ige wäßrige Lösung in Frage. Besonders bevorzugt ist der Fall, daß die ω-Amino-α-aminocarbonsäure Lysin ist. Im Prinzip sind solche Reaktionen aus Houben-Weyl 1977, Band XV/I, S. 468ff bekannt, doch eignet sich besonders vorteilhaft der Einsatz von Dimethylcarbonat (DMC), um die ω-Amino-α-aminosäure nur an seiner ω-Aminofunktion mit einer außerordentlich hohen Selektivität zu schützen.

Diese Schutzgruppen-Einführung kann wahlweise in einem zweiphasigen System aus Wasser und einem organischen Lösungsmittel, welches nicht mit Wasser mischbar ist, durchgeführt werden. In diesem Falle haben sich als organische Lösungsmittel aliphatische Kohlenwasserstoffe, chlorierte Kohlenwasserstoffe sowie aromatische Kohlenwasserstoffe oder Ether sowie Ketone bewährt. Als geeignete aliphatische Kohlenwasserstoffe sind zu nennen: n-Pentan, n-Hexan, n-Heptan, Cyclopentan, Cyclohexan, Methylcyclohexan, Petrolether, Ligroin.

Als chlorierte/bromierte Kohlenwasserstoffe besonders geeignet sind: Chloroform, Methylenchlorid, 1,1,1-Trichlorethan, 1,1,2-Trichlorethan, 1,2-Dichlorethan, Bromoform, Dibrommethan, 1,2-Dibromether.

Als aromatische Kohlenwasserstoffe geeignet sind: Toluol, 1,2-, 1,3-, 1,4-Xylol, Mesitylen.

Als Ether kommen in Frage: Diethylether, Methyl-tert.-butylether, 1,2-Diethoxyethylen.

Besonders bevorzugte Ketone sind: Methylisobutylketon, Diethylketon, Diisopropylketon, tert.-Butylmethylketon.

Eine vorteilhafte Ausgestaltung des o. g. Verfahrens bildet ebenfalls die Umsetzung von ω-Amino-α-aminosäuren oder eine diese Carbonsäuren erzeugende Vorstufe in einem einphasigen System aus Wasser mit dem Reagenz der allgemeinen Formel (IV), besonders vorteilhaft ist der Einsatz von DMC. Diesem System kann ggf. ein mit Wasser mischbares organisches Lösungsmittel zugesetzt werden. Als geeignete mit Wasser mischbare organische Lösungsmittel haben sich erwiesen: Alkohole, wie z. B. Methanol, Ethanol, Isopropanol, Propanol, tert.-Butanol, sec-Butanol, Isobutanol, Glykol. Weitere vorteilhafte mit Wasser mischbare organische Lösungsmittel sind Ether, wie z. B. THF, 1,2-Dimethoxyethan oder Diethylenglykol, Dioxan.

Bei Verwendung von DMC als Acylierungsreagenz bei o. g. Reaktion wird vorteilhafterweise mit einem Überschuß an DMC gearbeitet, so daß die eingesetzte ω-Amino-α-aminosäure bis zum maximal möglichen Grade ω-geschützt wird. Der Überschuß kann bis zum 10-fachen der equivalenten Menge an DMC verglichen mit der ω-Amino-α-aminosäure betragen. Besonders bevorzugt ist ein Überschuß von 3-5 eq.

Vorteilhaft ist, die ω-Amino-α-aminosäuren oder die diese Carbonsäuren erzeugenden Vorstufen als wäßrige basische Lösung vorzulegen und das DMC mit einem organischen Lösungsmittel verdünnt in die Lösung einzudosieren oder in Substanz zuzugeben. Bei Verwendung von organischen Verdünnungsmitteln entstehen dann, je nachdem, ob diese mit Wasser mischbar sind oder nicht, die oben beschriebenen ein- oder zweiphasigen Systeme. Der Einsatz von DMC pur ergibt auf jeden Fall ein einphasiges System.

Vorteilhafterweise sammelt sich beim Einsatz eines zweiphasigen Reaktionsmediums der nicht verbrauchte Überschuß des DMCs in der organischen oberen Phase und kann ohne die Gefahr der übermäßigen Verseifung in der wäßrigen basischen unteren Phase einfach durch Phasentrennung recycliert werden.

In jedem Fall wird, um die Verseifung des DMCs möglichst zu verringern und um kurze Reaktionszeiten und hohe Ausbeuten zu erhalten, vorteilhafterweise mit einer sehr guten Durchmischung der beiden Phasen gearbeitet. Diese entsprechend gute Vermischung kann durch besondere Rührorgane oder Pumpen, wie z. B. Seitenkanalpumpe, Jetmischer, Düsensysteme, Venturimischer oder Strahlsauger, etc., erreicht werden.

Bei Anwendung der einphasigen Arbeitsweise wird das DMC unter ebenfalls guter Durchmischung in die basische wäßrige Lösung der ω-Amino-α-aminosäure oder der diese Carbonsäure erzeugenden Vorstufe eingebracht. Es ist vorteilhaft, hierbei den Überschuß an DMC nach der Reaktion mit einem mit Wasser nicht mischbaren organischen Lösungsmittel extraktiv zu isolieren und zu recyclieren. Das so zurückgewonnene DMC kann erneut in einem nachfolgenden Arbeitszyklus eingesetzt werden.

Um die Konkurrenzreaktion zur Schutzgruppen-Einführung - die Hydrolyse - auf das maximal mögliche Maß zu reduzieren, wird die Reaktion der ω-Amino-α-aminosäure mit dem DMC in einem pH-Intervall von >8 und <14, bevorzugt ist 10-13, und ganz besonders bevorzugt ist 11-12, durchgeführt. Als Basen, welche zur Einstellung des pH-Wertes nützlich sind, können eingesetzt werden: Alkali-/Erdalkalimetallbasen oder Aminderivate. Bevorzugt dienen NaOH, KOH, Ca(OH)₂, Ba(OH)₂, Na₂CO₃, NaHCO₃, K₂CO₃, KHCO₃ oder NH₄OH, (NH₄)₂CO₃, NH₄HCO₃ zur pH-Kontrolle bei dieser Reaktion.

Ebenso sollten die Temperaturen der Umsetzung zwischen 5 °C und 25 °C, bevorzugt sind 10-20 °C, und ganz besonders bevorzugt bei 15 °C, liegen.

Die so erhaltene wäßrige ω-Moc-Amino-α-aminosäure-Lösung enthält neben dem Produkt der allgemeinen Formel (II) nur noch wenig Edukt sowie die Nebenprodukte Bis-Moc-(II) und α-Moc-(II) sowie eine gewisse Salzfracht. Trotzdem kann sie vorteilhafterweise ohne weitere Reinigungsschritte für den folgenden oxidativen Abbau eingesetzt werden.

Andere, sich von der allgemeinen Formel (IV) ableitbaren Acylierungsreagenzien können in äquivalenter Art zur Herstellung von ε-Alkoxycarbonyl-Lysin dienen.

Die Verbindungen der allgemeinen Formel (II) 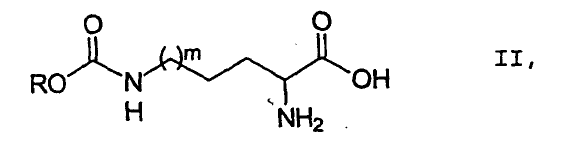 werden erfindungsgemäß in der oben erwähnten wäßrigen Lösung mit einem Oxidationsmittel aus der Gruppe der Halogene oder Halogenderivate zu Verbindungen der allgemeinen Formel (III) 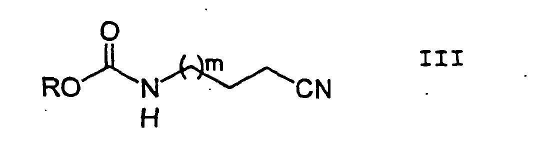 oxidativ decarboxyliert. Diese Reaktion ist prinzipiell aus der Literatur bekannt (Synth. Meth. 1982, 13, 548), sie wurde bisher jedoch nicht für die Umsetzung von Spezies der allgemeinen Formel (II) beschrieben. Als Halogene kommen für die Oxidation vorteilhafterweise die Verbindungen Chlor und Brom zum Einsatz. Geeignete Halogenderivate sind beispielsweise Hypochlorit, Chlorit, Trichlorisocyanurat, Dichlorisocyanurat, Chloramin-T, N-Chlorsuccinimid, 1,3-Dichlor-5,5-dimethylhydantoin, 1-Chlor-3-brom-5,5-dimethylhydantoin, N-Chlorsuccinimid, Hypobromit, Bromat/Bromid, Bromchlorid, 1,3-Dibrom-5,5-dimethylhydantoin, 1-Chlor-3-brom-dimethylhydantoin, N-Bromsuccinimid, Methylhypobromit, u. a. Besonders bevorzugt ist elementares Brom und die von Brom ableitbaren Oxidationsmittel, insbesondere BrCl und Gemische aus NaOCl und NaBr. Sie haben gegenüber Chlor bzw. den von Chlor abgeleiteten Oxidationsmitteln eine deutlich bessere Selektivität und neigen weit weniger zur Überoxidation.

Die Oxidation der Derivate (II) erfolgt vorteilhafterweise in wäßriger Lösung, die ggf. mit organischen Lösungsmitteln versetzt sein kann. Als organische Lösungsmittel kommen die oben angesprochenen, mit Wasser mischbaren Lösungsmittel in Frage.

Eine vorteilhafte Ausgestaltung des oben beschriebenen Verfahrens erfolgt dabei wie oben bereits beschrieben ohne Zwischenisolierung der Verbindung (II). Bei der Durchführung der Schutzgruppeneinführung in einem Zweiphasensystem wird nach der Reaktion die organische Phase von der wäßrigen getrennt. Die wäßrige Phase, in der (II) quantitativ vorliegt, kann ohne weitere Behandlung zur Oxidation herangezogen werden. Bei der Schutzgruppeneinführung in homogener Phase entfällt die nötige Phasentrennung. Hier kann nach Extraktion des DMCs gleich die Verbindung der allgemeinen Formel (II) mit den oben angegebenen Oxidationsmitteln umgesetzt werden. Die in den wäßrigen Phasen vorhandenen Nebenprodukte stören die Oxidation von (II) zur Verbindung (III) nicht. Es ist demgegenüber auch möglich und für das Erhalten eines möglichst reinen Produktes (III) vorteilhaft, wenn (II) aus seiner wäßrigen Produktphase durch Ansäuern als Feststoff isoliert und anschließend in einem separaten Schritt in einer wäßrigen Lösung mit Halogenen oder Halogenderivaten oxidiert wird.

Die Oxidation wird erfindungsgemäß zwischen -10 °C und 60 °C durchgeführt. Bevorzugt ist dabei der Bereich von 20-50 °C, ganz besonders bevorzugt der von 30-40 °C. Zur möglichst vollständigen Oxidation sind mindestens 2 eq. Oxidationsmittel notwendig (z. B. 2 mol Br₂ pro mol (II)). Bevorzugt wird mit einem Überschuß von 2-4 eq., besonders bevorzugt mit 2-3 eq. und ganz besonders bevorzugt mit 2-2,5 eq. gearbeitet.

Prinzipiell sind für die Oxidation zwei Varianten vorteilhaft. Bei der einen wird eine auf pH 11 bis 12 eingestellte wäßrige Lösung von (II) vorgelegt. In diese Lösung wird das Oxidationsmittel verdünnt oder unverdünnt eindosiert. Als Verdünnungsmittel eignen sich die oben angesprochenen Lösungsmittel, wie Alkohole oder chlorierte/bromierte Kohlenwasserstoffe, Ether und Wasser.

Eine andere besonders vorteilhafte Verfahrensweise geht davon aus, daß das Oxidationsmittel in verdünntem oder unverdünntem Zustand vorgelegt wird und eine auf pH 11 bis 12 eingestellte Lösung von (II) in diese zudosiert wird. Als Verdünnungsmittel für das Oxidationsmittel eignen sich wiederum die o. g. Lösungsmittel. Durch diese Verfahrensweise ist gewährleistet, daß zu jedem Zeitpunkt ein Überschuß an Oxidationsmittel vorhanden ist. Dadurch werden Nebenreaktionen, wie z. B. die Hydrolyse von Oxidationszwischenprodukten, zurückgedrängt, und die Produktausbeute wird maximal. Vorteilhafterweise liegt der pH-Wert bei der Oxidation im Bereich zwischen 6 und 13, bevorzugt ist ein Bereich von 7 bis 11, ganz besonders bevorzugt ist ein Bereich von 8-10.

Der soeben beschriebene Reaktionsschritt kann ggf. batchweise durchgeführt werden. Er stellt mithin für das Gelingen der Synthese von Verbindung (I) den kritischten Verfahrensabschnitt dar. Ganz besonders vorteilhaft erhält man bei dieser Oxidation dann sehr gute Ausbeuten, wenn das Oxidationsmittel und die zu oxidierende Spezies jeweils in einem bestimmten Verhältnis zueinander vorliegt. Dieses vorteilhafte Verhältnis vermindert das Ablaufen von unerwünschten Nebenreaktionen. Kommt z. B. das Endprodukt des oxidativen Abbaus (III) mit zuviel Oxidationsmittel in Berührung, dann resultiert ein erhöhtes Maß an Überoxidationsprodukten. Ist auf der anderen Seite während der Oxidation zuwenig Oxidationsmittel vorhanden, so wird die Weiterreaktion von Oxidationszwischenprodukten derart verlangsamt, daß diese im basischen Reaktionsmedium vermehrt hydrolysiert werden.

Eine vorteilhafte Lösung des oben geschilderten Problems bietet die Durchführung der Reaktion in Vorrichtungen oder Reaktionsmitteln, in denen ein ortsabhängiges Konzentrationsprofil eingestellt werden kann, wobei allerdings bei gegebenem Ort eine annähernd gleichbleibende Konzentration der Reaktanden existent ist, mithin diese also zeitlich konstant ist.

Dabei wird die zu oxidierende Spezies (II) in der wäßrigen Phase durch diese Vorrichtungen gepumpt und an einer oder mehreren Stellen das Oxidationsmittel zudosiert oder umgekehrt. Dadurch ist erfindungsgemäß gewährleistet, daß ein ortsabhängiges Konzentrationsprofil an Substrat und Reaktant vorliegt, welches durch die Eingangskonzentration an (II) und die Menge an zudosiertem Oxidationsmittel individuell und vorteilhaft für die Reaktion geregelt werden kann. Das Resultat ist, daß die Nebenproduktbildung minimal wird und die Ausbeuten an gewünschtem Nitril (III) im Optimalfall bei ca. 90 % oder darüber liegen. Die oben beschriebene Vorgehensweise ist besonders im großtechnischen Maßstab vorteilhaft, da hier durch die zusätzliche Regelmöglichkeit System-immanente Störungen, wie sie ein großtechnischer Batch-Prozeß unweigerlich mit sich bringt, abgefangen und gemildert werden können. Zudem kann die Reaktionswärme, welche bei diesem Prozeß ca. 700 kJ/mol beträgt, so bestens abgeführt werden, da die Energiefreisetzung in kleinen Einheiten während der gesamten Zeit über einen gewissen Weg geschieht. Man hat so die Möglichkeit des Einsatzes spezieller effizienterer Kühlkonzepte.

Ein weiterer, aber nicht minder wichtiger Vorteil dieser Art der Reaktionsführung ist die Möglichkeit der kontinuierlichen Fahrweise der Oxidation. Für die großtechnische Produktion von (I) bietet dies enorme Vorteile in Bezug auf die Raum/Zeit-Ausbeute und damit für dessen kostengünstige Herstellung.

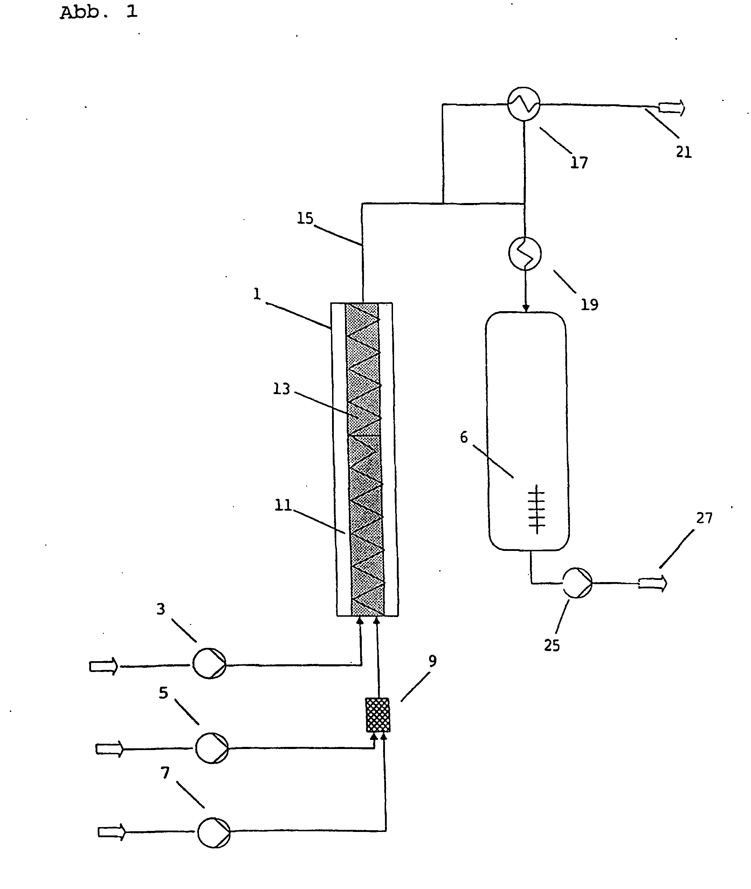

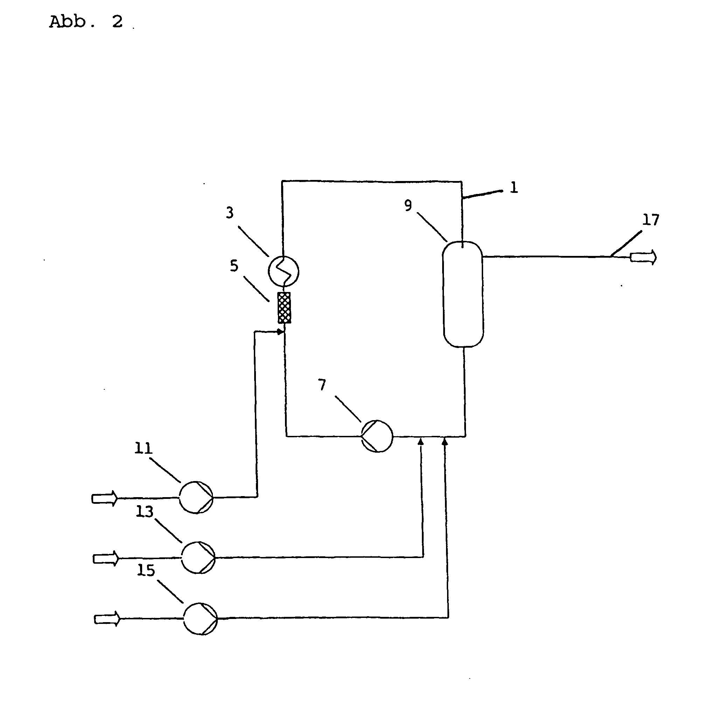

Prinzipiell sind alle dem Fachmann bekannten Vorrichtungen, in denen ein zeitlich konstantes, ortsabhängiges Konzentrationsprofil eingestellt werden kann, für diese Art von Reaktion zu verwenden (z. B. Rührkesselkaskade). Vorteilhaft ist der Einsatz eines Strömungsrohres bzw. eines Schlaufenreaktors. In Abb. 1 und Abb. 2 sind solche vorteilhaften Gefäße näher beschrieben.

An diese Oxidation anschließend wird die anfallende Reaktionslösung direkt mit Wasser nicht mischbaren organischen Lösungsmitteln extrahiert. Die Extraktion kann kontinuierlich oder diskontinuierlich durchgeführt werden. Da die anschließende Ringschlußreaktion unter Ausschluß von Wasser stattfinden muß, ist es besonders vorteilhaft zur Extraktion des Oxidationsprodukts (III) ein Lösungsmittel zu verwenden, welches sich azeotrop entwässern läßt. Ansonsten ist eine zusätzliche Trocknung des Lösungsmittels unumgänglich. Als besonders vorteilhafte organische Lösungsmittel haben sich Ethylacetat, Propylacetat, Butylacetat, Methylenchlorid, Toluol oder Gemische derselben erwiesen.

Die abschließende Ringschlußreaktion und die Überführung des Nitrils (III) 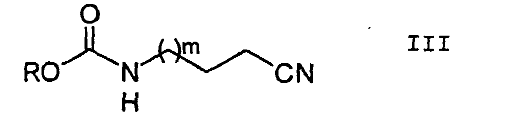 in ein Salz der Formel (I) 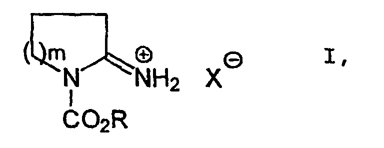 wird erfindungsgemäß mit einer anorganischen Säure durchgeführt. Ganz besonders bevorzugt ist der Einsatz von Chlorwasserstoff oder Bromwasserstoff. Erfindungsgemäß wird der Chlorwasserstoff oder Bromwasserstoff in einem bis zu 5-fachen molaren Überschuß eingesetzt. Besonders bevorzugt ist ein bis zu 4-facher Überschuß, ganz besonders bevorzugt ist ein 3-facher Überschuß an diesen Säuren. Zu beachten ist, daß, wenn das Substrat (III) in einem organischen Lösungsmittel vorliegt, dieses nach der azeotropen Entwässerung entweder vollständig entfernt wird und die Verbindung (III) in Substanz mit der Säure umgesetzt wird, oder das organische Lösungsmittel nach der azeotropen Entwässerung nur dann bei der Reaktion zugegen ist, wenn es gegen diese Säuren stabil ist. Besonders vorteilhafte Lösungsmittel sind deshalb solche, die sich sowohl azeotrop entwässern lassen sowie gegen Chlorwasserstoff und Bromwasserstoff inert sind. Ganz besonders bevorzugt ist deshalb der Einsatz von Toluol zur Extraktion und Ringschlußreaktion.

Bei der Verwendung von Toluol in Kombination mit Chlorwasserstoff oder Bromwasserstoff bilden sich bei der Reaktion zwei Phasen. In der oberen Phase ist Toluol mit etwas Chlorwasserstoff oder Bromwasserstoff gelöst vorhanden, während die untere Phase die Säure und das Produkt (I) enthält. Erfindungsgemäß wird nach der Zyklisierung zur Einleitung der Kristallisation ein Alkohol oder ein Ether zum Zyklisierungsgemisch zugegeben. Als Ether eignen sich hier insbesondere Methyl-tert.-butylether, 1,2-Dimethoxyethan oder THF, Dioxan. Als Alkohole werden bevorzugt Methanol, Ethanol, sec-Butanol, Isobutanol und ganz besonders bevorzugt Isopropanol eingesetzt. Vorteilhaft ist es aber auch, vor der Kristallisation eine Phasentrennung durchzuführen. Dabei wird die produkthaltige Säurephase zur Kristallisation in Ether oder Alkohole eingetragen.

Erfolgt die Umsetzung der Verbindung (III) in Substanz mit der anorganischen Säure, so wird nach Vollendung der Reaktion dieses Gemisch in die oben angegebenen Ether oder Alkohole gegeben bzw. mit diesen versetzt, um die Kristallisation zu initiieren.

Bei allen Kristallisationsvarianten ist es von Vorteil, den Anteil an anorganischer Säure in dem Reaktionsgemisch vor dem Versetzen mit Alkoholen oder Ethern mittels Vakuum zu verringern, um einen möglichst geringen Anteil der Säuren im Kristallisationslösungsmittel vorliegen zu haben. Dies erleichtert die Recyclierung dieser Lösungsmittel.

Bevorzugt wird deshalb vor oder während der Zugabe oder dem Vermengen der Reaktionsgemische mit Alkoholen oder Ethern ein Vakuum angelegt.

Vorteilhafte Ausgestaltungen der Zyklisierungsreaktion ergeben sich, wenn das Nitril (III), welches wasserfrei in Toluol gelöst vorliegt, mit der dreifachen stöchiometrischen Menge an Chlorwasserstoff versetzt wird. Das dabei resultierende zweiphasige Gemisch besteht im wesentlichen aus einer Toluolphase mit einem Chlorwasserstoffgehalt von ca. 1,5 bis 1,8 % und einer Chlorwasserstoffphase, welche im wesentlichen das Produkt enthält. Nach der Reaktion wird das resultierende zweiphasige Gemisch durch Anlegen eines Vakuums vom überwiegenden Teil des Chlorwasserstoffs befreit, wobei das Endprodukt langsam beginnt auszukristallisieren. Durch Zugabe von THF wird die Kristallisation beschleunigt und restlicher Chlorwasserstoff gelöst. Nach Abtrennen des Feststoffs mittels eines Filters wird der Filterkuchen erneut mit THF gewaschen. Der Filterkuchen wird anschließend bei 35-40 °C getrocknet.

Eine erfindungsgemäße Ausgestaltung dieses Verfahrens geht so vor, daß das zweiphasige Gemisch zunächst phasengetrennt wird. Die obere, im wesentlichen aus Toluol und ca. 1,5 bis 1,8 % HCl bestehende Phase kann für den Folgeansatz verwendet werden. Die untere, im wesentlichen aus Produkt und Chlorwasserstoff bestehende Phase wird unter Anlegen eines Vakuums direkt mit Isopropanol versetzt oder in Isopropanol eingeleitet. Dabei fällt das Hydrochlorid der Verbindung (I) aus. Diese Fällung kann entweder in der Art vorgenommen werden, daß die untere organische Phase unter leichter Kühlung bei ca. 15 °C in Isopropanol dosiert wird, oder das Isopropanol wird in die organische Phase einlaufen gelassen. Die Temperatur sollte in beiden Fällen 30 °C nicht überschreiten, um Ausbeuteverluste vorzubeugen. Der überwiegende Teil des Chlorwasserstoffs wird durch das vorhandene Vakuum entfernt. Bevor der Niederschlag mittels Filter oder Zentrifuge abgetrennt wird, wird die Suspension zunächst auf ca. 5 °C heruntergekühlt. Der Filterkuchen wird anschließend mit kaltem Isopropanol nachgewaschen und bei 35 bis 40 °C im Vakuum getrocknet. Zur Vervollständigung der Kristallisation können die vereinigten Isopropanol-Lösungen im Vakuum bei maximal 40 °C eingedampft werden. Wieder fällt etwas Produkt (I) aus, welches mit wenig frischem Isopropanol aufgeschlämmt und gewaschen wird. Die Ausbeute kann durch dieses Verfahren nochmals deutlich gesteigert werden. Ebenso wie das Toluol kann das abdestillierte Isopropanol ohne weitere Reinigung für den nächsten Batch verwendet werden.

Bei der Zyklisierung der Verbindung (III) zu (I) mit Chlorwasserstoff in Substanz kann die Kristallisation erfindungsgemäß wie eben beschrieben erfolgen. Die beschriebene Phasentrennung vom Toluol entfällt dann. Von Vorteil ist weiterhin die Möglichkeit der kontinuierlichen Reaktionsführung. Die ganz besondere Bedeutung dieser Aufarbeitungsvariante liegt darin, daß bei geeignet reinem Produkt eine Kristallisation des Salzes (I) aus einem Lösungsmittel ganz entfallen kann. In diesem Fall wird die produkthaltige Chlorwasserstoffphase einfach der Sprühtrocknung oder einer Wirbelschicht/bett-Trocknung zugeführt.

Das Auffinden einer geeigneten Methode zum selektiven Schützen der ω-Aminofunktion der ω-Amino-α-aminosäure sowie die Möglichkeit, den oxidativen Abbau des Derivats (II) zu (III) und dessen Ringschluß zu (I), gefolgt von der verbesserten Kristallisationsvariante in einer derart einfachen und für einen großtechnischen Prozeß vorteilhaften Weise durchführen zu können, ist mithin ursächlich dafür, das Salz (I) in Bezug auf ökologische wie ökonomische Gesichtspunkte gegenüber dem Verfahren des Standes der Technik in überlegener Weise herstellen zu können.

Unter einem linearen (C₁-C₄)-Alkylrest versteht man Methyl-, Ethyl-, n-Propyl- und den n-Butylrest.

Die folgenden Beispiele sollen die Erfindung beschreiben, sie jedoch keinesfalls einschränken.

### Beispiel 1:

### Batchweise Herstellung von N-(4-Cyanobutyl)-carbaminsäuremethylester

Eine wäßrige Lösung von 1055 g MOC-Lysin in Form einer 15,42%igen Roh-Lösung wird im Kolben vorgelegt. Innerhalb von 1 h wird bei einer Temperatur von max. 35 °C 319,6 g elementares Brom eindosiert. Parallel hierzu wird der pH-Wert durch Dosierung von 533 g 30%iger NaOH auf pH = 9,6 gehalten. Nach Beendigung der Dosierung wird 15-30 Minuten bei gleicher Temperatur nachgerührt und anschließend auf Raumtemperatur abgekühlt. Die wäßrige Phase wird dreimal mit je 100 ml Ethylacetat extrahiert. Die vereinigten Extrakte werden anschließend destillativ i. Vak. bei einer Sumpftemperatur von < 50 °C aufgearbeitet. Es werden 115,6 g N-(cyanobutyl)-carbaminsäuremethylester in einer Reinheit von 87 % erhalten. Dies entspricht einer Ausbeute von 81 %.

### Beispiel 2:

### Batchweise Herstellung von N-(4-Cyanobutyl)-carbaminsäuremethylester

Eine Lösung von 2086 g Brom in 9275 g Dichlormethan wird im Kolben vorgelegt und auf ca. 10 °C abgekühlt. Es wird mit der Dosierung (1 l/h) von Natronlauge begonnen. In Summe werden ca. 2302 g (26,4 mol) NaOH in Form einer 50%igen wäßrigen Lösung dosiert. Kurze Zeit später (ca. 5 Minuten) wird 1/3 der MOC-Lysin Menge parallel dazu dosiert, während die Temperatur durch Kühlung auf 10 °C gehalten wird. Das zweite Drittel MOC-Lösung kann bei 15 °C und das dritte Drittel bei 25 °C dosiert werden. In Summe werden innerhalb von 2 h 1225,38 g (6 mol) MOC-Lysin in Form einer ca. 11-12%igen Roh-Lösung dosiert. Nach Dosierung der Komponenten wird 1 h bei 25 °C nachgerührt. Ausgefallenes Salz wird abfiltriert und mit ca. 3600 g Dichlormethan nachgewaschen. Das zweiphasige System wird anschließend intensiv gerührt, um das Produkt zu extrahieren. Nach Phasentrennung wird die wäßrige Phase noch zweimal mit je 5 l Dichlormethan extrahiert. Die vereinigten Extrakte werden anschließend i. Vak. bei < 50 °C Sumpftemperatur eingedampft. Man erhält 915 g N-(Cyanobutyl)-carbaminsäuremethylester mit einem Gehalt von 87 %. Dies entspricht einer Ausbeute von 85 %.

### Beispiel 3:

55,7 kg (30 mol) Moc-Lysin werden als 11%ige wäßrige Lösung bei max. 25 °C mit einer Lösung aus 83,7 kg (96 mcl) Natriumchlorit als ca. 14,25%ige Lösung und 10,3 kg (100 mol) Natriumbromid in 20 1 Wasser gelöst versetzt. Der pH-Wert wird dabei mittels konz. Natronlauge auf 9,7 bis 10,0 gehalten. Nach 10 min werden 1,1 kg Natriumhydrogensulfit zur Entfernung des überschüssigen Oxidationsmittels zur Reaktionslösung gegeben und diese anschließend 3 mal mit 40 l Essigester extrahiert. Der Extrakt enthielt 3,78 kg N-(4-Cyanobutyl)-carbaminsäuremethylester (80,7 %).

### Beispiel 4:

### Kontinuierliche Herstellung von N-(4-Cyanobutyl)-carbaminsäuremethylester (III) im Strömungsrohr

Abb. 1 zeigt ein Strömungsrohr 1 mit Zu- und Abläufen. Die Zuläufe werden über die Pumpen 3, 5 und 7 geregelt. Optional können die Zuläufe der Pumpen 5 und 7 mittels eines statischen Mischers 9 gekoppelt werden und anschließend in das Strömungsrohr 1 münden. Das Strömungsrohr 1 besteht aus einem Mantelkühler 11 und ist mit einer Füllkörpereinlage 13 gepackt. An das Strömungsrohr 1 schließt sich ein Ablauf 15 an. Dieser Ablauf 15 verzweigt sich zu einem Wärmetauscher 17 und einem Wärmetauscher 19. Der Wärmetauscher 17 wiederum steht via Rohrleitung mit dem Wärmetauscher 19 in Kontakt und hat einen separaten Abfluß 21. Der Wärmetauscher 19 ist einem Puffergefäß 23 vorgeschaltet. Nach dem Puffergefäß 23 befindet sich eine Pumpe 25 und ein Ablauf 27.

Über die Pumpe 3 werden stündlich 1 mol bzw. 0,5 mol s-Moc-Lysin in das Strömungsrohr eindosiert. Optional kann über die Pumpen 5/7 Brom und Natronlauge entweder direkt in das Strömungsrohr oder vor der Einspeisung über einen statischen Mischer 9 vorgemischt eingespeist werden. Der pH im Strömungsrohr beträgt dabei 9,0-10. Die Temperatur im Strömungsrohr wird mittels Kühler 11 auf ca. 40 °C eingestellt. Anschließend wird das Reaktionsgemisch der Extraktion über den Ablauf 15 zugeführt.
a)

| MOC-LYS (mol/h) | Brom (mol/h) | ÜS Brom (eq.) | T (°C) | pH | VZ (min) | Selektivität (%) | MOC-LYS (%) |
|---|---|---|---|---|---|---|---|
| 1 | 4,5 | 2,25 | 40 / 48 | 9,1-9,3 | 8 | 80 | 17 |

b)

| MOC-LYS (mol/h) | Brom (mol/h) | ÜS Brom (eq.) | T (°C) | pH | VZ (min) | Selektivität (%) | MOC-LYS (%) |
|---|---|---|---|---|---|---|---|
| 0,5 | 2,5 | 2,5 | 35 / 40 | 9,0 | 6 | 82 | 7,4 |
| 0,5 | 2,5 | 2,5 | 35 / 40 | 9,5 | 6 | 91 | 6,7 |
| 0,5 | 2,5 | 2,5 | 35 / 40 | 10,0 | 6 | 90 | 6,2 |
| 0,5 | 2,5 | 2,5 | 34 / 33 | 9,8 | 2,8 | 79 | 24,8 |
| a) mit statischem Mischer 9 b) ohne statischen Mischer 9 VZ: Verweilzeit [min] ÜS: Überschuß | | | | | | | |

### Beispiel 5:

### Kontinuierliche Herstellung von N-(4-Cyanobutyl)-carbaminsäuremethylester (III) in einer Reaktionsschlaufe

Abb. 2 zeigt einen Schlaufenreaktor mit An- und Abflüssen. Der Schlaufenreaktor besteht aus einem ringförmigen Rohrsystem 1. In das Rohrsystem 1 integriert ist ein Wärmetauscher 3. Dem Wärmetauscher 3 nachgeschaltet ist ein statischer Mischer 5, welchem eine Pumpe 7 und ein Überlaufgefäß 9 folgt. Das Überlaufgefäß 9 ist wieder mit dem Wärmetauscher 3 mittels eines Rohrsystems 1 verbunden. Über einer Pumpe 11 existiert ein Zulauf zwischen dem Mischer 5 und der Pumpe 7. Zwei Zuläufe von den Pumpen 13 und 15 bestehen zwischen der Pumpe 7 und dem Überlaufgefäß 9. Das Überlaufgefäß 9 enthält einen Abfluß 17.

In den Schlaufenreaktor werden stündlich 1,64 1 ε-Moc-Lysin als 9 %ige wäßrige Lösung über die Pumpe 11 eindosiert. Dazu werden 0,54 l 20 %ige NaOH über die Pumpe 13 und 0,060 ml Br₂ über die Pumpe 15 eingespeist. Die Reaktionstemperatur liegt bei ca. 35-40 °C.

| MOC-LYS (mol/h) | Brom (mol/h) | ÜS Brom (eq.) | T (°C) | pH | VZ (min) | Selektivität (%) | MOC-LYS (%) |
|---|---|---|---|---|---|---|---|
| 0,75 | 4,5 | 3 | 37-38 | 9,4-9,6 | 6,6 | 88,3 | 3,6 |
| ÜS: Überschuß VZ: Verweilzeit [min] | | | | | | | |

### Beispiel 6:

### Herstellung von MOC-Lysin

### a) Zweiphasigkeit

3654 g (20 mol) Lysin*HCl werden in 9000 ml Wasser gelöst und in einem 30 l Rührbehälter vorgelegt. Mit 50 %iger Natronlauge wird ein pH von ca. 11,6 eingestellt und die Lösung anschließend auf 14-15 °C abgekühlt. Anschließend werden bei 14-15 °C 5406 g (60 mol) Dimethylcarbonat und 3000 ml Toluol zugesetzt und dann intensiv mit dem Rühren begonnen. Nach 7-8 h unter den obigen Bedingungen ist die Reaktion beendet. Während der kompletten Reaktionszeit wird der pH-Wert durch Dosieren einer 50 %igen Natronlauge konstant auf 11,6 gehalten. Hierzu waren ca. 1770 g (10,77 mol) 30 %ige Natronlauge erforderlich.

Zur Aufarbeitung wird der Rührer angestellt. Nach ca. 30 Minuten haben sich die organische und die wäßrige Phase voneinander getrennt. Die untere wäßrige Phase enthält das Produkt (91 %), unumgesetztes Lysin (8 %) und wenig Nebenprodukte (1 %), die obere organische Phase besteht im wesentlichen aus Toluol und unumgesetztem Dimethylcarbonat (2620 g = 52 %). Dies entspricht einem DMC-Verlust durch Verseifung von ca. 38 %. Die Dimethylcarbonat-haltige Toluol-Phase wird mit frischem Dimethylcarbonat ergänzt und ohne weitere Manipulation für den nächsten Ansatz verwendet. Die untere wäßrige Phase wird ohne weitere Manipulation für den oxidativen Abbau eingesetzt.

### b) Einphasigkeit

In einem 2 l Rührkolben werden 372 g (2,0 mol) Lysinhydrochlorid in 900 g Wasser vorgelegt. Durch Zugabe von 152 g (3,8 mol) Natronlauge wird ein pH-Wert von 11,6 eingestellt. Nach Abkühlen auf 13-15 °C werden 541 g (6,0 mol) Dimethylcarbonat zugesetzt und die Reaktion durch intensives Rühren gestartet. Die Reaktion ist nach 7 h bei dieser Temperatur beendet. Während der kompletten Reaktionszeit wurde der pH-Wert durch kontinuierliches Dosieren von 430 ml Natronlauge (10 %ig) konstant bei 11,5-11,7 gehalten.

Die wäßrige Phase enthält ca. 90,5 % MOC-Lysin, 8,5 % Lysin und ca. 1 % NP. Das überschüssige Dimethylcarbonat war vollständig verseift worden.

### Beispiel 7:

### Zyklisierung von N-(4-Cyanobutyl)-carbaminsäuremethylester zu N-Methoxyiminopiperidin

a)
   31,2 g (0,2 mol) N-(4-Cyanobutyl)-carbaminsäuremethylester (85 %ig) werden in 165 ml Toluol bei RT gelöst. Anschließend wird die Lösung auf 15 °C gekühlt und bei dieser Temperatur 21,9 g (0,6 mol) Chlorwasserstoff 1 h lang eingeleitet. Es wird 1 h bei 15-20 °C nachgerührt, wobei ein Zweiphasensystem entsteht. Zur Aufarbeitung wird zunächst der Überschuß an Chlorwasserstoff bei RT und einem Vakuum von bis zu 100 mbar entfernt. Mit beginnender Kristallisation werden 83 ml THF zur vollständigen Ausfällung des Produktes zugesetzt.
   Das ausgefallene Produkt wird abgetrennt und ein weiteres Mal mit 83 ml THF gewaschen, um die verbliebenen Chlorwasserstoff-Reste zu entfernen.
   Das Produkt wird abgetrennt und bei 35-40 °C i. Vak. (10 mbar) getrocknet.
   Man erhält nach dem Trocknen 34,7 g (90 %) N-Methoxyiminopiperidinsalz in Form eines leicht gelblichen Feststoffs.
b)
   Zunächst wird analog Beispiel 7a) verfahren. Nach Beendigung der Nachreaktionszeit werden die beiden Phasen getrennt. Die obere Toluol-Phase enthält noch ca. 1,5-1,8 % Chlorwasserstoff. Diese Phase kann ohne weitere Manipulation für die nächste Zyklisierung eingesetzt werden.
   Die untere Produktphase wird in 85 ml Isopropanol dosiert, wobei das Produkt ausfällt. Parallel zur Dosierung der Produktphase wird ein Vakuum bis zu 100 mbar angelegt, um den Überschuß an Chlorwasserstoff zu entfernen.
   Das Produkt wird abgetrennt, mit 50 ml Isopropanol nachgewaschen und i. Vak. (10 mbar) bei 35-40 °C getrocknet.
   Man erhält nach dem Trocknen 35,6 g (92,3 %) N-Methoxyiminopiperidinhydrochlorid in Form eines weißen Feststoffs.
c)
   156 g (1,0 mol) N-(4-Cyanobutyl)-carbaminsäuremethylester werden in einem Kolben vorgelegt und bei 15-20 °C 109,5 g (3,0 mol) Chlorwasserstoff innerhalb von 3 h eingeleitet. Es wird 1 h bei 15-20 °C nachgerührt. Anschließend läßt man die Reaktionslösung in 300 ml Isopropanol laufen, das zuvor auf 0-5 °C abgekühlt wurde. Dabei fällt ein hell gelber Niederschlag aus. Die Suspension wird auf 25-30 °C erwärmt. Bei dieser Temperatur wird mittels Vakuum innerhalb von 30 Minuten der Überschuß an Chlorwasserstoff entfernt. Bevor das Produkt isoliert wird, wird die Suspension auf 0-5 °C abgekühlt. Das isolierte Produkt wird mit 200 ml kaltem Isopropanol nachgewaschen und i. Vak. (10 mbar) bei 35-40 °C getrocknet.
   Man erhält nach dem Trocknen 175,4 g (91,1 %) N-Methoxycarbonyliminopiperidinhydrochlorid in Form eines weißen Feststoffs.
d)
   Man verfährt wie in Beispiel 7c beschrieben. Anschließend wird allerdings der Chlorwasserstoff im Vakuum entfernt und der verbleibende Rest im Trockenschrank bei 50 °C getrocknet.
   Man erhält 200,1 g (97,1 %, 93,46%ig) N-(4-Cyanobutyl)-carbaminsäuremethylester.

### Beispiel 8:

### Herstellung von ε-Methoxycarbonyl-ornithin

211 g (1,25 mol) Ornithinhydrochlorid werden in einem 2 l Rührbehälter mit 400 ml Wasser vorgelegt. Unter leichter Kühlung werden 400 g einer 25%igen NaOH zudosiert. Anschließend werden bei 15 °C 340 g (3,75 mol) Dimethylcarbonat gelöst in 300 ml Toluol zugegeben. Durch kontinuierliche Dosierung von Natronlauge wird der pH-Wert über die komplette Reaktionszeit von 7 h konstant bei pH = 11,5 gehalten (Verbrauch an 25%-iger NaOH über die gesamte Reaktionszeit 205 ml).
Nach Beendigung der Reaktion werden die Phasen getrennt. Die obere Toluol-Phase (378 g) enthält noch ca. 18 % DMC (69 g). Die untere wäßrige Produktphase enthält noch ca. 2,83 % Ornithin (39,2 g), was einem Umsatz von 81,4 % entspricht.
Diese wäßrige Lösung kann problemlos dem oxidativen Abbau zum N-(Cyanopropyl)-carbaminsäuremethylester zugeführt werden.
Zum Isolieren des MOC-Ornithins wird der pH-Wert mittels Salzsäure auf pH = 4,2 eingestellt, wobei das Produkt ausfällt. Nach den Trocken bei 40 °C i. Vak. erhält man 199 g MOC-Ornithin mit einem Gehalt von 87,8 % (enthält 8,9 % NaCl). Dies entspricht einer Isolierausbeute von 80,7 %.

### Beispiel 9:

### Herstellung von N-(Cyanopropyl)-carbaminsäuremethylester

221,2 g (1,0 mol) MOC-Ornithin-Na-salz werden in Form einer 10,56%-igen wäßrigen Lösung analog Beispiel 4 umgesetzt. Im Betriebszeitraum werden, unter sonst identischen Bedingungen, zusätzlich 400 g Brom (2,5 mol) und ca. 5 mol NaOH in Form einer 25%-igen wäßrigen Lösung zudosiert.
Es werden 3438 g Reaktionslösung erhalten. Die Reaktionslösung wird mit Essigester extrahiert. Nach destillativer Entfernung des Extraktionsmittels erhält man 158 g Produkt (95,6%-ig). Die Ausbeute ist quantitativ.

### Beispiel 10:

### Herstellung von 1-Methoxycarbonyl-2-iminopyrrolidinhydrochlorid

In 155 g (1,04 mol) N-(Cyanopropyl)-carbaminsäuremethylester in 200 ml Toluol werden bei 20 °C 77 g Chlorwasserstoff unter leichter Kühlung eingeleitet. Dabei fällt das Produkt schon nach kurzer Zeit aus. Es wird wie üblich aufgearbeitet. Man erhält 180 g Produkt. Dies entspricht einer Ausbeute von 93 %.

## Patentansprüche

1. Verfahren zur Herstellung eines Salzes der allgemeinen Formel (I) 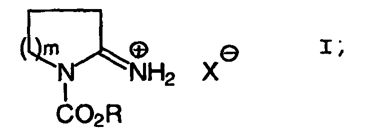 wobei X^{⊖} ein anorganisches Anion, m = 1, 2 oder 3 und R einen linearen (C₁-C₄)-Alkylrest darstellt,
**dadurch gekennzeichnet,**
**daß** man
a) eine entsprechende ω-Amino-α-aminosäure selektiv an seiner ω-Aminofunktion zu Verbindungen der allgemeinen Formel (II) umsetzt 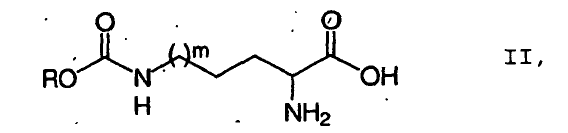
b) die Verbindung der allgemeinen Formel (II) mit einem Oxidationsmittel aus der Gruppe der Halogene oder Halogenderivate oder Gemische derselben zu Verbindungen der allgemeinen Formel (III) oxidiert 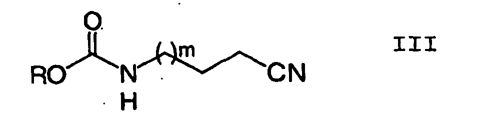
c) und die Verbindung der allgemeinen Formel (III) unter Wasserausschluß in Gegenwart einer Säure durch Ringschluß zum Salz der allgemeinen Formel (I) cyclisiert.

2. Verfahren nach Anspruch 1,
**dadurch gekennzeichnet,**
**daß** man die entsprechende ω-Amino-α-aminosäure oder eine diese Carbonsäure erzeugende Vorstufe unter basischen Bedingungen mit einem Reagenz der allgemeinen Formel (IV) 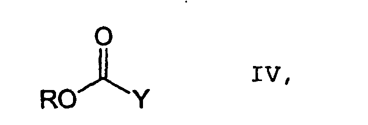 worin Y = Cl, Br oder OMe oder 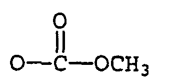 ist und R einen linearen (C₁-C₄)-Alkylrest oder Arylrest darstellt, zu Verbindungen der allgemeinen Formel (II) umsetzt.

3. Verfahren nach Anspruch 2,
**dadurch gekennzeichnet,**
**daß** die ω-Amino-α-aminosäure Lysin ist.

4. Verfahren nach Anspruch 2 und/oder 3,
**dadurch gekennzeichnet,**
**daß** man in einem zweiphasigen System aus Wasser und einem organischen Lösungsmittel, welches nicht mit Wasser mischbar ist, arbeitet.

5. Verfahren nach Anspruch 2 und/oder 3,
**dadurch gekennzeichnet,**
**daß** man in einem einphasigen System aus Wasser und ggf. einem mit Wasser mischbaren organischen Lösungsmittel arbeitet.

6. Verfahren nach Anspruch 2 bis 5,
**dadurch gekennzeichnet,**
**daß** man das Reagenz der allgemeinen Formel (IV) in einem Überschuß von 1-10 equiv., bevorzugt sind 3-5 equiv., bezogen auf die verwendete ω-Amino-α-aminosäure einsetzt.

7. Verfahren nach Anspruch 2 bis 6,
**dadurch gekennzeichnet,**
**daß** die Umsetzung bei einem pH-Wert von >8 und <14, bevorzugt ist 10-13, erfolgt.

8. Verfahren nach Anspruch 2 bis 7,
**dadurch gekennzeichnet,**
**daß** die Temperatur bei der Umsetzung 5 °C bis 25 °C, bevorzugt sind 10 °C bis 20 °C, beträgt.

9. Verfahren nach Anspruch 1 und 2
**dadurch gekennzeichnet,**
**daß** man die Verbindung der allgemeinen Formel (II) in wäßriger Lösung ggf. unter Zusatz von organischen Lösungsmitteln oxidiert.

10. Verfahren nach Anspruch 9,
**dadurch gekennzeichnet,**
**daß** man die Verbindung der allgemeinen Formel (II) vor der Oxidation als Feststoff isoliert.

11. Verfahren nach Anspruch 9,
**dadurch gekennzeichnet,**
**daß** man die Verbindung der allgemeinen Formel (II) ohne Zwischenisolierung oxidiert.

12. Verfahren nach Anspruch 1 und 2 und 9 bis 11,
**dadurch gekennzeichnet,**
**daß** die Temperatur bei der Umsetzung zwischen -10 °C und 60 °C, bevorzugt ist 20-50 °C, liegt.

13. Verfahren nach Anspruch 1 und 2 und 9 bis 12,
**dadurch gekennzeichnet,**
**daß** man das Oxidationsmittel in einem Überschuß von 2-4 equiv., besonders bevorzugt 2-3 equiv. und ganz besonders bevorzugt 2-2,5 equiv., bezogen auf das Substrat der allgemeinen Formel (II) einsetzt.

14. Verfahren nach Anspruch 1 und 2 und 9 bis 13,
**dadurch gekennzeichnet,**
**daß** der pH-Wert bei der Oxidation zwischen 6 und 13, bevorzugt 7-11 und ganz besonders bevorzugt 8-10,

15. Verfahren nach Anspruch 1 und 2 und 9 bis 14,
**dadurch gekennzeichnet,**
**daß** man die Umsetzung batchweise durchführt.

16. Verfahren nach Anspruch 1 und 2 und 9 bis 14,
**dadurch gekennzeichnet, daß** man die umsetzung in einem Schlaufenreaktor oder einem Strömungsrohr durch führt.

17. Verfahren nach Anspruch 1 und 2
**dadurch gekennzeichnet,**
**daß** man die Verbindung der allgemeinen Formel (III) mittels einer anorganischen Säure in das Salz der allgemeinen Formel (I) überführt.

18. Verfahren nach Anspruch 17,
**dadurch gekennzeichnet,**
**daß** die anorganische Säure Chlorwasserstoff oder Bromwasserstoff ist.

19. Verfahren nach Anspruch 18,
**dadurch gekennzeichnet,**
**daß** man die Säure in einem Überschuß von bis zu 5 equiv. bezogen auf das Substrat der allgemeinen Formel (III), bevorzugt sind 2-3 equiv., einsetzt.

20. Verfahren nach Anspruch 17 bis 19,
**dadurch gekennzeichnet,**
**daß** man die Verbindung der allgemeinen Formel (III) in einem mit Wasser nicht mischbaren organischen Lösungsmittel gelöst mit der Säure versetzt.

21. Verfahren nach Anspruch 17 bis 19,
**dadurch gekennzeichnet,**
**daß** man die Verbindung der allgemeinen Formel (III) in Substanz mit der Säure versetzt.

22. Verfahren nach Anspruch 17 bis 21,
**dadurch gekennzeichnet,**
**daß** man die Umsetzung bei 10 °C bis 30 °C, bevorzugt ist 15 °C bis 20 °C, durchführt.

23. Verfahren nach Anspruch 20,
**dadurch gekennzeichnet,**
**daß** man das Salz der allgemeinen Formel (I) durch Zugabe von Ethern und/oder Alkoholen zum Reaktionsgemisch kristallisiert.

24. Verfahren nach Anspruch 20 oder 21,
**dadurch gekennzeichnet,**
**daß** man die produkthaltige Säurephase zur Kristallisation von (I) mit Ethern oder Alkoholen vermengt.

25. Verfahren nach Anspruch 23 oder 24,
**dadurch gekennzeichnet,**
**daß** man vor oder während der Zugabe oder dem Vermengen mit Alkoholen und/oder Ethern ein Vakuum anlegt.

26. Verfahren nach Anspruch 21,
**dadurch gekennzeichnet,**
**daß** man die produkthaltige Chlorwasserstoffphase der Sprühtrocknung oder einer Wirbelschicht/bett-Trocknung zuführt.

## Claims

1. Process for the production of a salt of the general formula (I) 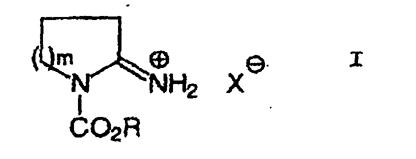 wherein X^{⊖} represents an inorganic anion, m = 1, 2 or 3, and R represents a linear (C₁-C₄)-alkyl radical,
**characterized in that**
a) a corresponding ω-amino α-amino acid is reacted selectively at its ω-amino function to give compounds of the general formula (II) 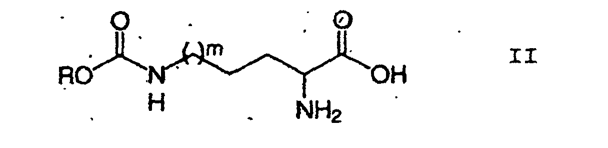
b) the compound of the general formula (II) is oxidized with an oxidizing agent from the group of the halogens or halogen derivatives or mixtures thereof to give compounds of the general formula (III) 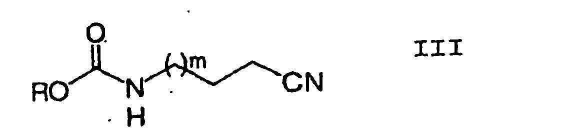
c) and the compound of the general formula (III) is cyclized with exclusion of water in the presence of an acid by ring closure to give the salt of the general formula (I).

2. Process according to Claim 1,
**characterized in that**
the corresponding ω-amino α-amino acid or a precursor which generates this carboxylic acid is reacted under basic conditions with a reagent of the general formula (IV) 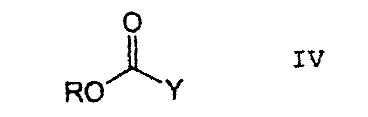 in which Y = Cl, Br or OMe or 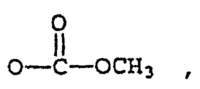 and R represents a linear (C₁-C₄)-alkyl radical or aryl radical to give compounds of the general formula (II).

3. Process according to Claim 2,
**characterized in that**
the ω-amino α-amino acid is lysine.

4. Process according to Claim 2 and/or 3,
**characterized in that**
the reaction of said ω-amino α-amino acid is conducted in a two-phase system prepared from water and a water-immiscible organic solvent.

5. Process according to Claim 2 and/or 3,
**characterized in that**
the reaction of said ω-amino α-amino acid is conducted in a single-phase system prepared from water and optionally a water-miscible organic solvent.

6. Process according to Claim 2 to 5,
**characterized in that**
said reagent of the general formula (IV) is used in an excess of 1-10 equiv., preferably 3-5 equiv., based on the ω-amino α-amino acid used.

7. Process according to Claim 2 to 6,
**characterized in that**
said reaction occurs at a pH of >8 and <14, preferably 10-13.

8. Process according to Claim 2 to 7,
**characterized in that**
the temperature in said reaction is 5°C to 25°C, preferably 10°C to 20°C.

9. Process according to Claim 1 and 2,
**characterized in that**
said compound of the general formula (II) is oxidized in aqueous solution, optionally with addition of organic solvents.

10. Process according to Claim 9,
**characterized in that**
said compound of the general formula (II) is isolated as a solid before the oxidation.

11. Process according to Claim 9,
**characterized in that**
said compound of the general formula (II) is oxidized without intermediate isolation.

12. Process according to Claim 1 and 2 and 9 to 11
**characterized in that**
the temperature in said reaction is between -10°C and 60°C, preferably 20-50°C.

13. Process according to Claim 1 and 2 and 9 to 12,
**characterized in that**
said oxidizing agent is used in an excess of 2-4 equiv., more preferably 2-3 equiv. and most preferably 2-2.5 equiv., based on the substrate of the general formula (II).

14. Process according to Claim 1 and 2 and 9 to 13,
**characterized in that**
the pH in said oxidation is between 6 and 13, preferably 7-11 and most preferably 8-10.

15. Process according to Claim 1 and 2 and 9 to 14,
**characterized in that**
said reaction is carried out batchwise.

16. Process according to Claim 1 and 2 and 9 to 14,
**characterized in that**
said reaction is carried out in a loop reactor or a flow tube.

17. Process according to Claim 1 and 2,
**characterized in that**
the compound of the general formula (III) is converted into the salt of the general formula (I) by means of an inorganic acid.

18. Process according to Claim 17,
**characterized in that**
said inorganic acid is hydrogen chloride or hydrogen bromide.

19. Process according to Claim 18,
**characterized in that**
said acid is used in an excess of up to 5 equiv. based on the substrate of the general formula (III), preferably 2-3 equiv.

20. Process according to Claim 17 to 19,
**characterized in that**
said acid is added to the compound of the general formula (III) dissolved in a water-immiscible organic solvent.

21. Process according to Claim 17 to 19,
**characterized in that**
said acid is added to the compound of the general formula (III) in substance.

22. Process according to Claim 17 to 21,
**characterized in that**
said reaction is conducted at 10°C to 30°C, preferably 15°C to 20°C.

23. Process according to Claim 20,
**characterized in that**
the salt of the general formula (I) is crystallized by addition of ethers and/or alcohols to the reaction mixture.

24. Process according to Claim 20 or 21,
**characterized in that**
the product-containing acid phase is mixed with ethers or alcohols for the crystallization of (I).

25. Process according to Claim 23 or 24,
**characterized in that**
a vacuum is applied before or during the addition or the mixing with alcohols and/or ethers.

26. Process according to Claim 21,
**characterized in that**
the product-containing hydrogen chloride phase is spray-dried or dried in a fluidized bed.

## Revendications

1. Procédé pour la préparation d'un sel de formule générale (I) 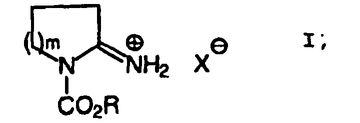 où X^{⊖} représente un anion inorganique, m = 1, 2 ou 3 et R représente un radical alkyle en C₁ à C₄, **caractérisé en ce que**
a) on transforme un ω-amino-α-aminoacide correspondant sélectivement sur sa fonction ω-amino en composés de formule générale (II) 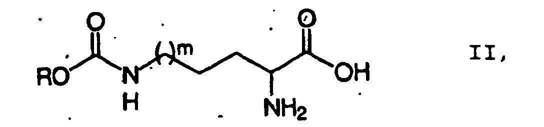
b) on oxyde le composé de formule générale (II) avec un oxydant du groupe des halogènes ou des dérivés halogénés ou des mélanges de ceux-ci en composés de formule générale (III) 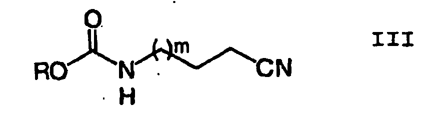
c) et on cyclise le composé de formule générale (III) à l'abri de l'eau en présence d'un acide par fermeture de cycle en sel de formule générale (I).

2. Procédé selon la revendication 1, **caractérisé en ce qu'**on transforme le ω-amino-α-aminoacide correspondant ou un précurseur produisant cet acide carboxylique dans des conditions basiques avec un réactif de formule générale (IV) 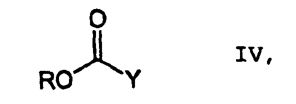 dans laquelle Y = Cl, Br ou OMe ou 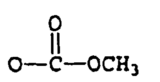 et R représente un radical alkyle en C₁ à C₄ linéaire ou un radical aryle, en composés de formule générale (II).

3. Procédé selon la revendication 2, **caractérisé en ce que** le ω-amino-α-aminoacide est la lysine.

4. Procédé selon la revendication 2 et/ou 3, **caractérisé en ce qu'**on travaille dans un système à deux phases constitué d'eau et d'un solvant organique, qui n'est pas miscible à l'eau.

5. Procédé selon la revendication 2 et/ou 3, **caractérisé en ce qu'**on travaille dans un système à une phase constitué d'eau et le cas échéant d'un solvant organique miscible à l'eau.

6. Procédé selon la revendication 2 à 5, **caractérisé en ce qu'**on utilise le réactif de formule générale (IV) en un excès de 1 à 10 équivalents, on préfère de 3 à 5 équivalents, par rapport au ω-amino-α-aminoacide.

7. Procédé selon la revendication 2 à 6, **caractérisé en ce que** la transformation est réalisée à un pH de >8 et <14, de préférence de 10-13.

8. Procédé selon la revendication 2 à 7, **caractérisé en ce que** la température lors de la transformation est de 5°C à 25°C, on préfère 10°C à 20°C.

9. Procédé selon la revendication 1 et 2, **caractérisé en ce qu'**on oxyde le composé de formule générale (II) en solution aqueuse, le cas échéant avec addition de solvants organiques.

10. Procédé selon la revendication 9, **caractérisé en ce qu'**on isole le composé de formule générale (II) avant l'oxydation sous forme solide.

11. Procédé selon la revendication 9, **caractérisé en ce qu'**on oxyde le composé de formule générale (II) sans isolement intermédiaire.

12. Procédé selon la revendication 1 et 2 et 9 à 11, **caractérisé en ce que** la température lors de la transformation est située entre -10°C et 60°C, on préfère 20-50°C.

13. Procédé selon la revendication 1 et 2 et 9 à 12, **caractérisé en ce qu'**on utilise l'oxydant en un excès de 2-4 équivalents, de manière particulièrement préférée de 2-3 équivalents, de manière tout particulièrement préférée de 2-2,5 équivalents, par rapport au substrat de formule générale (II).

14. Procédé selon la revendication 1 et 2 et 9 à 13, **caractérisé en ce que** le pH lors de l'oxydation est compris entre 6 et 13, de préférence 7-11 de manière tout particulièrement préférée 8-10.

15. Procédé selon la revendication 1 et 2 et 9 à 14, **caractérisé en ce qu'**on réalise la transformation par lots.

16. Procédé selon la revendication 1 et 2 et 9 à 14, **caractérisé en ce qu'**on réalise la transformation dans une colonne à bulles à écoulement en boucle ou un tuyau d'écoulement.

17. Procédé selon la revendication 1 et 2, **caractérisé en ce qu'**on transforme le composé de formule générale (III) au moyen d'un acide inorganique en sel de formule générale (I).

18. Procédé selon la revendication 17, **caractérisé en ce que** l'acide inorganique est l'acide chlorhydrique ou l'acide bromhydrique.

19. Procédé selon la revendication 18, **caractérisé en ce qu'**on utilise l'acide en un excès allant jusqu'à 5 équivalents par rapport au substrat de formule générale (III), on préfère 2-3 équivalents.

20. Procédé selon la revendication 17 à 19, **caractérisé en ce qu'**on mélange le composé de formule générale (III) sous forme dissoute dans un solvant organique non miscible à l'eau avec l'acide.

21. Procédé selon la revendication 17 à 19, **caractérisé en ce qu'**on mélange le composé de formule générale (III) en masse avec l'acide.

22. Procédé selon la revendication 17 à 21, **caractérisé en ce qu'**on réalise la transformation à 10°C jusqu'à 30°C, on préfère de 15°C à 20°C.

23. Procédé selon la revendication 20, **caractérisé en ce qu'**on cristallise le sel de formule générale (I) par addition d'éthers et/ou d'alcools au mélange réactionnel.

24. Procédé selon la revendication 20 ou 21, **caractérisé en ce qu'**on mélange la phase acide contenant le produit en vue de la cristallisation du composé (I) avec des éthers ou des alcools.

25. Procédé selon la revendication 23 ou 24, **caractérisé en ce qu'**on met sous vide avant ou pendant l'addition ou le mélange avec des alcools et/ou des éthers.

26. Procédé selon la revendication 21, **caractérisé en ce qu'**on introduit la phase d'acide chlorhydrique contenant le produit dans un séchage par pulvérisation ou dans un séchage en couche/lit fluidisé.
